# EUROPEAN PATENT APPLICATION

(11) **EP 4 442 871 A1**
(43) Date of publication of application: **09.10.2024**
(21) Application number: 22901406.3
(22) Date of filing: 01.12.2022
(51) Int. Cl.: C40B 40/10, C07K 5/12, C07K 7/54, C07K 7/64, C07K 17/00, G01N 33/545

(54) **PEPTIDE-IMMOBILIZED BEAD LIBRARY**

(30) Priority: 03.12.2021 JP 2021197219
(71) Applicant: HiPep Laboratories, Kyoto 602-8158 (JP)
(72) Inventor: NOKIHARA, Kiyoshi, Kyoto-shi, Kyoto 602-8158 (JP); SASAKI, Toru, Kyoto-shi, Kyoto 602-8158 (JP); KASAMA, Takeshi, Kyoto-shi, Kyoto 602-8158 (JP)
(74) Representative: Mewburn Ellis LLP
(86) International application number: PCT/JP2022/044358
(87) International publication number: WO 2023/100976

(57) **Abstract**

Disclosed is a peptide-immobilized bead library from which peptides are more likely to be obtained as candidates of useful substances such as pharmaceuticals, and with which the amino acid sequence bound to a target substance can be more easily analyzed, compared to known OPOB libraries. The peptide-immobilized bead library has a structure of General Formula [I]. 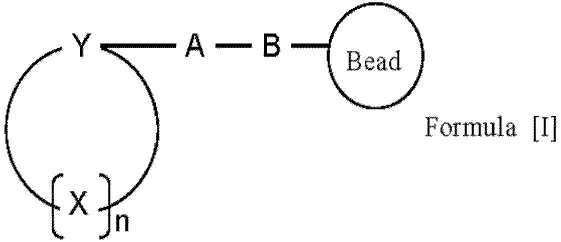 In General Formula [I], X is an amino acid residue; n is an integer of 4 to 8; Y is a cyclization structure; A is a spacer structure; B is a specific cleavage site-containing structure; and the 4 to 8 amino acid residues of X are each independently randomly selected.

## Description

### TECHNICAL FIELD

The present invention relates to a peptide-immobilized bead library used, for example, to search for an amino acid sequence that binds to a target substance.

### BACKGROUND ART

In methods of searching for an amino acid sequence that binds to a target substance, peptides are immobilized on beads, and the peptide-immobilized beads are brought into contact with the target substance, followed by analyzing the amino acid sequence of a peptide immobilized on a bead to which the target substance is bound. Such a process includes immobilizing only one kind of peptide per bead to prepare a peptide library containing a large number of beads, and bringing the peptide library into contact with a target substance. The method in which only one kind of peptide is immobilized per bead to prepare a peptide library is called OPOB (one peptide immobilized on one bead), and has been used for drug discovery.

The present inventors discovered that the recognition ability and the stability of the peptides in OPOB can be enhanced by use of cyclic peptides as the peptides to be immobilized on the beads, and confirmed that the use of cyclic peptides also enables accurate analysis, as reported previously (Non-Patent Document 1). Non-Patent Document 1 describes a peptide-immobilized bead having a structure of the following formula.

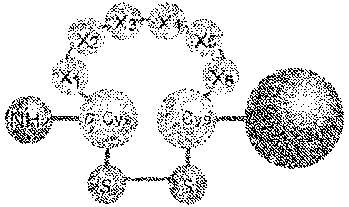

In the above formula, X₁ to X₆ are arbitrary amino acid residues independent of each other. D-Cys is bound to each of both ends of the six amino acid residues, and the peptide is cyclized by an S-S bond between the D-Cys at both ends. In the above formula, the large sphere at the right end is a bead.

### PRIOR ART DOCUMENT

### [Non-Patent Document]

[Non-Patent Document 1] Kiyoshi Nokihara et al., Amino Acids (2016) 48:2491-2499

### SUMMARY OF THE INVENTION

### PROBLEMS TO BE SOLVED BY THE INVENTION

The OPOB library described in Non-Patent Document 1 exerts an excellent recognition ability due to the cyclization of the peptides. However, even in cases where a peptide that has contacted and bound to (recognizes) a target substance is obtained, the peptide often has physical properties undesirable as a drug candidate. Further, after sorting the bead that has contacted and bound to the target substance, the amino acid sequence of the peptide immobilized on the bead needs to be analyzed. However, the analysis is not always easy, and requires a complicated operation and an expensive apparatus.

Thus, an object of the present invention is to provide a peptide-immobilized bead library from which peptides are more likely to be obtained as candidates of useful substances such as pharmaceuticals, and with which the amino acid sequence bound to the target substance can be more easily analyzed, compared to the OPOB library described in Non-Patent Document 1.

### MEANS FOR SOLVING THE PROBLEMS

As a result of intensive study, the present inventors inferred that peptides that bind to target substances can be newly obtained by binding peptides having combinations of amino acids selected based on conventional results, to beads through spacers. The present inventors also inferred that, in the analysis of the amino acid sequence of each peptide, the amino acid sequence of the peptide can be more easily analyzed by cleaving off the peptide from the bead, and succeeded in providing a specific cleavage site that can be easily and specifically cleaved between the peptide and the bead, thereby reaching the present invention.

Specifically, the present invention provides the following.
(1) A peptide-immobilized bead library having a structure of the following General Formula [I]: 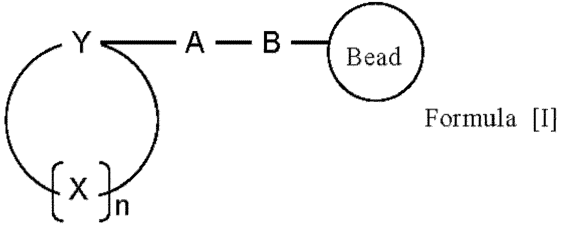 (wherein in General Formula [I], X is an amino acid residue; n is an integer of 4 to 8; Y is a cyclization structure; A is a spacer structure; B is a specific cleavage site-containing structure; and the 4 to 8 amino acid residues of X are each independently randomly selected).
(2) The peptide-immobilized bead library according to (1), wherein the Y is selected from a disulfide bond, a thioether bond, and an amide bond.
(3) The peptide-immobilized bead library according to (2), wherein the Y is a structure containing a disulfide bond, and constituted by two cysteine residues.
(4) The peptide-immobilized bead library according to (2), wherein the Y is a structure containing a thioether bond, and constituted by a homocysteine or cysteine, and an acetyl group.
(5) The peptide-immobilized bead library according to any of (1) to (4), wherein the B is a methionine residue.
(6) The peptide-immobilized bead library according to any of (1) to (5), wherein the A is a structure containing 3 to 30 atoms constituting the main chain of the A.
(7) The peptide-immobilized bead library according to any of (1) to (6), wherein
   the 4 to 8 amino acid residues of X are each independently randomly selected from a group of amino acid residues; and
   half or more of the amino acid residues constituting the group are amino acid residues other than the 20 kinds of amino acid residues constituting naturally occurring proteins.

### EFFECT OF THE INVENTION

By the present invention, a novel peptide-immobilized bead library that securely achieves binding to a target substance, and that enables easier analysis of the amino acid sequence that has bound to the target substance, was provided.

### BRIEF DESCRIPTION OF THE DRAWINGS

Fig. 1 is a diagram illustrating a result of mass spectrometry obtained in the Examples below.
Fig. 2 is a diagram illustrating a result of mass spectrometry obtained in the Examples below.

### MODE FOR CARRYING OUT THE INVENTION

As described above, the peptide-immobilized bead library of the present invention is a peptide-immobilized bead library of the General Formula [I].

In the General Formula, X is an amino acid residue, and n is an integer of 4 to 8. This means that the number of the amino acid residues immobilized on one bead is 4 and 8. The number of the amino acid residues is preferably 5 to 7, especially preferably 6.

The amino acids that give the 4 to 8 amino acid residues are each independently randomly selected. It is well known that 20 kinds of amino acids constitute naturally occurring proteins (the 20 kinds of amino acids may be hereinafter referred to as "protein-constituting amino acids", and amino acids other than the 20 kinds of amino acids may be hereinafter referred to as "non-protein-constituting amino acids", for convenience). Examples of the amino acids used in the present invention include not only the protein-constituting amino acids, but also a variety of non-protein-constituting amino acids.

The amino acids that give X in General Formula [I] are selected from a group of amino acids. In cases where the number of amino acids constituting the group of amino acids (which may be hereinafter referred to as "amino acid building units" for convenience) is large, the number of kinds of amino acid sequences is extremely large as a result, so that the synthesis of an OPOB covering all amino acid sequences is extremely laborious. In Non-Patent Document 1, peptides were constituted by selecting 6 amino acids from 24 kinds of amino acid building units, which correspond to 24⁶ (about 200 million) amino acid sequences. Therefore, careful and repetitive dividing and mixing were necessary, and much effort was required in the synthesis operation. Thus, there is a trade-off relationship between the diversity of the amino acid sequences and the amount of work for the synthesis, and, taking the commercial applicability into account, an appropriate number of kinds of amino acid building units is thought to be about 10 to 14, preferably about 11 to 13.

In cases of drug discovery, the group of amino acid building units is preferably designed such that new drugs can be discovered at higher probability. The Lipinski's rule, often used in drug discovery, shows the following general trends in oral pharmaceuticals:
1. not more than 5 hydrogen bond donors (NH, OH);
2. not more than 10 hydrogen bond acceptors (N, O);
3. a molecular weight of not more than 500; and
4. a distribution coefficient of not more than 5 in terms of LogP.

The Lipinski's rule is known to be partially consistent even in exceptional cases. For example, the cyclic peptide cyclosporine has a molecular weight of 1207, but has five hydrogen bond donors. In addition, it is generally desirable to have fewer free-rotating moieties, and this is thought to be important from the viewpoint of the affinity and the specificity. The present inventors carefully examined past reports in an attempt to discover peptide drugs comprising amino acid units that frequently appear. In general, aromatic amino acids make significant contributions. Amino acids containing a functional group at the end of a long methylene chain are less likely to be selected since they are entropically disadvantageous. Also at the interacting interface of antibodies, Tyr makes a significant contribution, and tends to cause less free rotation.

As a result of further study, the present inventors inferred the following criteria for the selection of the amino acid building units:
1. the presence of a side chain that is representative as a pharmacophore (aromatic, aliphatic, anionic, or cationic), or high likeliness to contribute to diversification of the main chain structure;
2. low free rotation between a functional group that serves as a pharmacophore, and the main chain (for example, Dap is preferred to Lys, and Phe is preferred to HomoPhe);
3. lowest possible hydrogen bond-forming ability (although hydrogen bonds are advantageous to interactions in some cases, their contribution is generally small in the primary hit, so that priority is given to not impairing physical properties);
4. distinguishability from other amino acids based on the molecular weight (since the enantiomers D-isomer and L-isomer are indistinguishable by mass spectrometry, the number of sets of D-isomer and L-isomer was reduced to accept up to three pairs based on the premise that identification is carried out by single-product synthesis after the sorting of the beads; since primary structures obtained by the search are commonly modified in the art for the purpose of maximization or optimization, the D- or L-isomer may be introduced at the time of such modification); and
5. relatively high availability of non-naturally occurring amino acid derivatives as commercially available products (also taking into account the cost of industrial production from the viewpoint of their practical application).

In addition, the following points were taken into account as conditions obtained from the result of a selective experiment using a display system.
1. Non-protein-constituting amino acids are used to increase the amino acid building units in order to achieve structural diversity with high quality (D-amino acids or non-naturally occurring side chains are introduced taking into account their high contribution to the affinity).
2. Peptides having a certain extent of drug-like properties should be obtained even in cases where they have moderate affinities (although the Lipinski's rule is not always applicable to drug discovery, the rule is taken into account to some extent).
3. The screening is carried out using a target expressed in human cells.
4. Identification of unknown target molecules is also carried out as another obj ect.

Among these, the use of non-protein-constituting amino acids to increase the amino acid building units, mentioned in 1, is especially important, and half or more of the amino acids constituting the immobilized peptides are preferably non-protein-constituting amino acids. This increases the probability of discovery of new drugs that have not been discovered so far compared to cases where the protein-constituting amino acids are mainly used.

Further, as described later, since the amino acid sequence of the immobilized peptide is preferably analyzed by mass spectrometry, it is preferred not to include amino acid building units having the same molecular weight in the same group. However, since even leucine and isoleucine, which have the same molecular weight, can be distinguished from each other by high energy collision-induced dissociation using MALDI-TOF-MS/MS, amino acid building units having the same molecular weight may also be included in the same group.

Taking these points into account, the following Groups 1 to 3 were designed as preferred amino acid building unit groups.

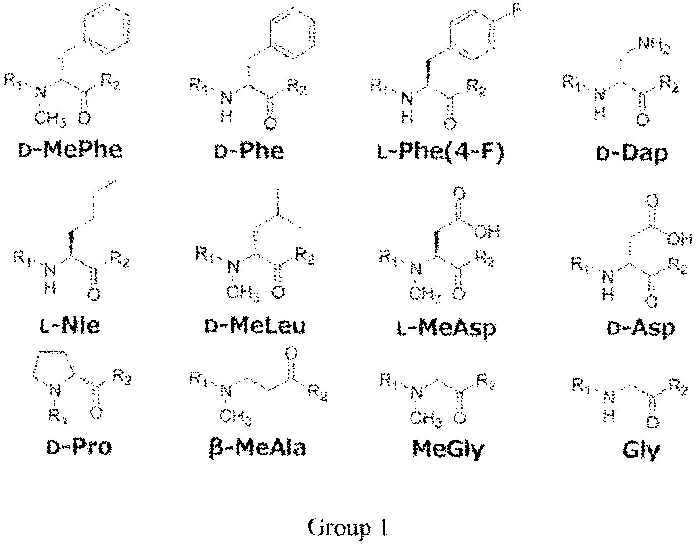

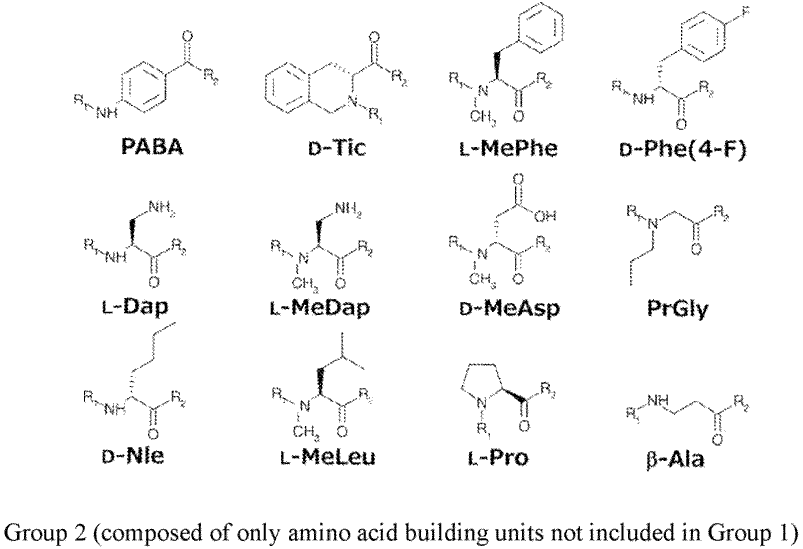

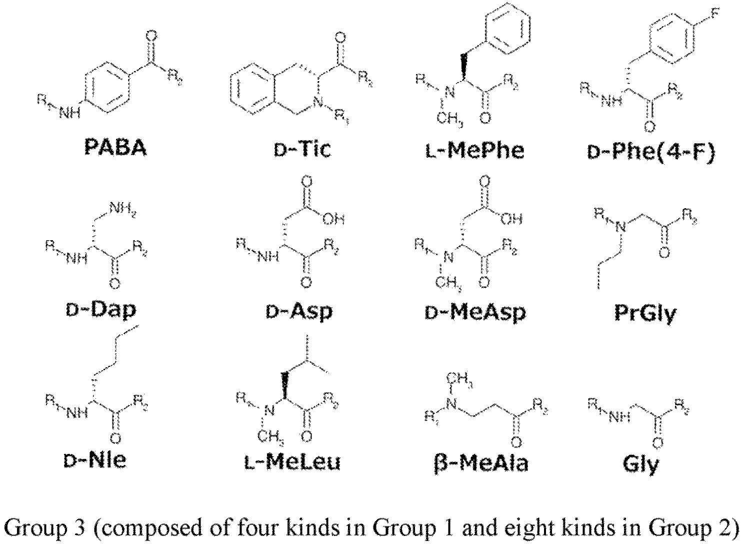

The group of amino acid building units is not limited to Group 1 to Group 3. Other groups may also be employed in accordance with the criteria described above.

The 4 to 8 amino acid residues described above are cyclized by the cyclization structure Y as shown in General Formula [I]. Cyclization of peptides in a peptide-immobilized bead library is known as already described in Non-Patent Document 1, and the known method may be employed as it is. More specifically, cysteine (which may be L-cysteine, but is preferably D-cysteine from the viewpoint of suppressing enzymatic degradation *in vivo)* may be bound to each of both ends of the peptide composed of the 4 to 8 amino acid residues, and the cysteines may be bound to each other through an S-S bond, to achieve the cyclization. However, the cyclization structure is not limited to the two cysteines, and may be any structure as long as the cyclization can be achieved. For example, homocysteine may be bound to one end of the peptide, and a halogenated acetyl group may be bound to the other end, followed by performing cyclization through a thioether bond formed by binding of the homocysteine to the acetyl group. Alternatively, the cyclization may be carried out through an amide bond formed by binding of an amino group to a carboxyl group.

In General Formula [I], A is a spacer structure. The spacer structure ensures that the binding reaction between the target substance and the immobilized peptide is not disturbed by steric hindrance by the bead, and the structure itself is not limited. The number of atoms constituting the main chain of the spacer structure is preferably 3 to 30, more preferably 5 to 15. In its simplest form, the spacer structure may be an alkylene group having 3 to 30 carbon atoms which optionally contains another atom. In cases where the spacer structure is an alkylene group, the spacer structure can be simply formed using aminoalkylene acid, which has a carboxyl group at one end and an amino group at the other end. For example, in the Examples below, a spacer structure was formed using 6-aminohexyl acid (Ahex) between a methionine residue as a specific cleavage site (described later) and cysteine. However, the spacer structure is not limited thereto.

In General Formula [I], B is a specific cleavage site-containing structure. The specific cleavage site-containing structure is not limited as long as the structure has a site that can be specifically cleaved. Preferably, the structure is simple, and has a site that can be specifically cleaved by the action of a chemical. Preferred specific examples of the specific cleavage site-containing structure include methionine. Methionine is specifically cleaved by cyanogen bromide (BrCN).

Since a variety of beads having a functional group on the surface are commercially available, a commercially available bead may be used as the bead in General Formula [I]. The size of the bead is not limited. Since the bead bound to the target substance can be preferably collected by a micropipette, the bead preferably has a size at which observation under an optical microscope is possible. The size is preferably about 50 µm to 100 µm in diameter. Preferred specific examples of the bead include, but are not limited to, TentaGel S-NH₂ (trade name) (a polystyrene bead grafted with polyethylene glycol (PEG), having an amino group on the surface and a diameter of 90 µm), which is commercially available.

The peptide-immobilized bead library of the present invention can be produced as follows. First, a specific cleavage site-containing structure such as methionine is bound using a functional group on the bead surface. Subsequently, a spacer structure (such as the Ahex used in the Examples) is bound. Further, predetermined amino acids are bound by a conventional method such as the Fmoc method. Cyclization is then carried out by a disulfide bond or a thioether bond. This method can be carried out by conventional methods of organic synthetic chemistry and peptide synthesis, and is specifically described in the Examples below. Further, the methods *per se* of sequential binding of amino acids and cyclization of a peptide are known as described in Non-Patent Document 1, and also specifically described in the Examples below.

The method of using the peptide-immobilized bead library of the present invention is the same as the method of using the known OPOB library described in Non-Patent Document 1 except that a step of cleaving the specific cleavage site-containing structure is included. More specifically, first, a target substance is brought into contact with the peptide-immobilized bead library. After washing, beads to which the target substance is bound are selectively collected. This operation can be carried out using a micropipette by observation under an optical microscope. In cases where the target substance is a cell, the cell is visible under an optical microscope. Therefore, beads to which cells are bound are collected using a micropipette. In cases where the target substance is a molecule such as a protein, the target substance itself cannot be seen under an optical microscope. Therefore, the target substance is labeled. In cases where, for example, a fluorescent dye is used as the label, beads to which a substance emitting fluorescence is bound are collected using a micropipette under irradiation with light that causes the emission of the fluorescence.

Subsequently, the specific site in the specific cleavage site-containing structure is cleaved to cleave off the bead. In cases where, for example, the specific cleavage site-containing structure is methionine, this cleavage can be carried out by the action of BrCN. The reaction can be carried out, for example, under conditions at a final BrCN concentration of 20 mM to 300 mM in a solvent such as 50 mM to 1 M hydrochloric acid, at a temperature of 4°C to 60°C, for 30 minutes to 24 hours.

After the cleavage reaction, the bead is removed. The removal of the bead can be carried out, for example, by taking out the supernatant using a pipette.

After the removal of the bead, the amino acid sequence of the cyclic peptide in the structure cleaved off from the bead is determined. This *step per se* can be carried out by any known method such as a method described in Non-Patent Document 1. For example, the step can be carried out by Edman degradation, which is a conventional method. However, for the sequence analysis, a method using mass spectrometry is preferred because of its flexibility since, unlike Edman degradation, this method does not require the long analysis time for the sequential cleavage, and does not require a free N-terminal amino group in the sample. For the sequence analysis, the method commonly known as MS/MS is useful. In MS/MS, two mass spectrometers (MS) are connected in series, and a collision activation cell is placed between them. The principle of the method is as follows. First, the sample is ionized in the first MS, and then only ions having a particular mass number are selected and guided into the collision activation cell to cause their collision with an inert gas, followed by measuring, in the second MS, secondary ions (product ions) generated from the ions selected in the first MS. The analysis can be simply carried out using advanced software.

In cases where a selective experiment is carried out for molecules such as free proteins, molecules having interaction with the cyclic peptides can also be identified by a mass spectrometer. More specifically, a solution that promotes protein denaturation and modification reaction such as reductive alkylation is mixed with the beads to release interacting proteins, and then limited hydrolysis is carried out using an enzyme or the like, followed by identification of the proteins using a mass spectrometer. On the other hand, cyclic peptides left covalently bound to the beads are specifically cleaved off from the beads by the above-described method, and then their sequences are analyzed using a mass spectrometer. The present method for detecting the interacting molecules as well as the library may be carried out for a large number of beads, and use of such a system allows application of the present invention also to an attempt for comprehensive search for proteins or the like that interact with a group of peptides having structures that are likely to be candidate substances of pharmaceuticals.

The present invention is concretely described below based on Examples. However, the present invention is not limited to the following Examples.

Example 1

### (1) Method of Synthesizing OPOB Having Particular Peptide Sequence

As the beads (TentaGel S NH2, 90 µm), Fmoc amino acids, coupling agent (HATU), deprotection reagent, organic solvents, and the like to be used for the peptide synthesis, products manufactured by HiPep Laboratories, Nacalai Tesque Inc., Novabiochem (Merck Millipore), or BLD Pharm were used.

An OPOB having a particular peptide sequence was synthesized as follows. First, beads in an amount equivalent to 10 µmol were weighed and taken into a reaction vessel RT5 (High-Pep Laboratories), and dimethylformamide (DMF) was added thereto to cause swelling of the beads, followed by placing the reaction vessel in a solid-phase synthesizer PetiSyzer PSS510 (HiPep Laboratories). In a polytube, a solution of the Fmoc amino acid corresponding to the C-terminal residue (60 µmol) in DMF was mixed with HATU (60 µmol) and diisopropylethylamine (DIEA, 120 µmol), and the resulting mixture was added to the beads, followed by stirring the resulting mixture for 45 minutes at room temperature. The beads were washed five times with DMF, and DMF containing 20% piperidine was added thereto, followed by stirring the resulting mixture for 5 minutes. After repeating this reaction twice, the beads were washed again five times with DMF. The extension cycle, composed of the coupling reaction for the Fmoc amino acid and the Fmoc removal reaction with piperidine, was repeated in accordance with the sequence, and then acetylation was carried out by the same method, followed by washing the obtained beads twice with DMF, five times with dichloromethane, five times with methanol, and five times with t-butylmethylether. The beads were then dried using a vacuum pump. A mixed solution of trifluoroacetic acid, trimethylsilane, and water (90:5:5) was ice-cooled and added to the beads, and the resulting mixture was stirred for 90 minutes at room temperature. When methionine was included in the sequence, 30 equivalents of tetra-n-butylammonium bromide was added, and the resulting mixture was stirred for additional 5 minutes. The beads were washed five times with dichloromethane, and six times with a mixture of water and an organic solvent. When a disulfide bond was to be formed, the beads were stirred for 24 hours in an aqueous solution containing 0.1 M ammonium acetate and 10% dimethyl sulfoxide (DMSO). When a thioether bond was to be formed, the beads were stirred for 1 hour in an aqueous solution containing 0.2 M DIEA and 20% DMSO. In either case, the beads were subsequently washed six times with a mixture of water and an organic solvent, to obtain peptide-immobilized beads (OPOB).

### (2) Synthesis of OPOB Library

For synthesis of a medium-scale OPOB library (corresponding to 480 µmol of beads), first, a common part was linked by the same chemical reaction as that for the particular sequence using a reaction vessel RTG50 (High-Pep Laboratories) and a solid-phase synthesizer PetiSyzer II (HiPep Laboratories). Fmoc was then deprotected at the position before the amino acid randomization site. The resulting suspension was divided into 12 portions such that the amount of beads was almost the same among the portions, and the portions were transferred into 12 RT5s. In a polytube, a solution of each Fmoc amino acid (160 µmol) in DMF was mixed with HATU (160 µmol) and DIEA (320 µmol) to prepare a solution, and the prepared solution was added to the beads (in an amount corresponding to 40 µmol) in each RT5 on PetiSyzer PSS510, followed by stirring the resulting mixture at 40°C for 45 minutes. The resulting 12 kinds of bead suspensions were combined in one RTG50, and washed five times with DMF. Similarly, thereafter, coupling reaction was carried out after dividing the beads into 12 RT5s, and washing and Fmoc removal reaction were carried out after mixing the beads in one RTG50, to allow extension of the amino acids at the randomization site. Finally, linking of a common part was carried out in an RTG50. The deprotection reaction and the formation of the disulfide bond or thioether bond were carried out in the same manner as in the preparation of the OPOB of the particular sequence.

### Example 2 Sequence Analysis of OPOB by Mass Spectrometer

### (1) Sequence Analysis of Disulfide-Bonded Cyclic OPOB

One bead was placed in an AHST vial (H255, HiPep Laboratories), and liquid was removed as much as possible. The bead was washed once with 10 µL of water. After removal of the water, the bead was reduced with 10 µL of 0.83 mM TCEP (tricarboxyethyl phosphine) (at 40°C for 1 hour). After removal of the TCEP, the bead was alkylated with 10 µL of 10 mM IAM (iodoacetamide) (at 40°C for 45 minutes under light-shielded conditions). The IAM was then removed.

To the reduced and alkylated bead, 10 microliter (µL) of 0.1 M hydrochloric acid containing cyanogen bromide (final concentration, 50 mM) was added, and the resulting mixture was left to stand at room temperature for 2 hours.

The solution after the cleavage of the peptide was taken out, and dried using a centrifugal evaporator. The peptide was then redissolved in 10 µL of 0.1% TFA. After placing 1 µL of the solution on a MALDI plate, 1 µL of CHCA (5 mg/mL in 50% TFA) was added thereto, followed by drying. MS and MS/MS spectra were measured by ultrafleXtreme.

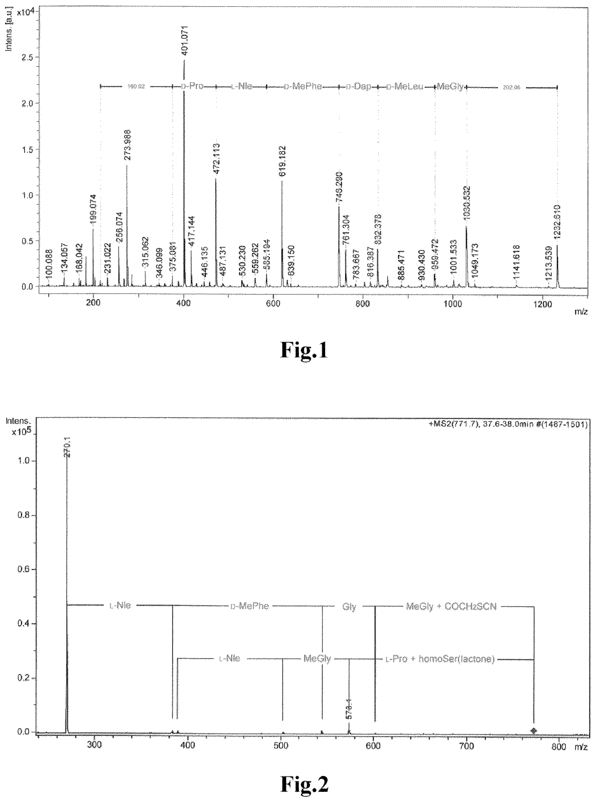

The original sequence before the cyanogen bromide treatment, in the state where Cys was reduced, was Ac-D-Cys-MeGly-D-MeLeu-D-Dap-D-MePhe-L-Nle-D-Pro-D-Cys-Ahx-Met-Beads. The peptide immobilized on the one bead contained a Met residue, which was converted to homoserine lactone by the CNBr treatment. Fig. 1 shows the result of mass spectrometry of Ac-D-Cys-MeGly-D-MeLeu-D-Dap-D-MePhe-L-Nle-D-Pro-D-Cys-Ahx-homoSer(lactone), which was subjected to the mass spectrometry.

### (2) Sequence Analysis of Thioether-Bonded Cyclic OPOB

One bead was placed in an AHST vial, and liquid was removed as much as possible. The bead was washed once with 10 µL of water. After adding 10 µL of 4% BrCN in ACN/AcOH/H2O to the bead, the reaction was allowed to proceed at 40°C for 3 hours.

The solution after the cleavage of the peptide was taken out, and dried using a centrifugal evaporator. The peptide was then redissolved in 20 µL of 0.1% formic acid. After injecting 10 µL of the resulting solution into LC/MS, MS and MS/MS spectra were measured.

The original sequence before the cyanogen bromide treatment was [Ac-MeGly-Gly-D-MePhe-L-Nle-MeGly-L-Pro-homoCys] -Ahx-Met-Beads (wherein the structure in [] is a structure cyclized by a thioether bond), and the original immobilized peptide on the bead thus contained the thioether-type cyclic body and the Ahex-Met residue. Fig. 2 shows the result of mass spectrometry of NC-S-CH2CO-MeGly-Gly-D-MePhe-L-Nle-MeGly-L-Pro-homoSer(lactone), which was subjected to the mass spectrometry.

### Screening Using OPOB (General Examples)

The following is a general description of methods of screening using OPOB.

A method of sorting (screening for) beads that recognize a target molecule from an OPOB library varies depending on how the target molecule is expressed or what detection method is employed. However, in any case, the method can be carried out by allowing the library to interact with the target molecule in an aqueous solution having an appropriate composition, and then collecting beads whose binding to the target molecule is found. For the purpose of obtaining only highly specific beads, it is also effective to employ, for example, a method in which the collected beads are allowed to interact with molecules other than the target molecule or with cells of non-target lineages, and then beads bound to the molecules or cells are excluded from the analysis, or a method in which more stringent washing conditions are employed by changing the composition of the aqueous solution in which the interaction is allowed to occur.

In cases where a target molecule that can be hardly solubilized is used for the screening as a protein expressed outside the cell membrane, or where a structure of a molecule or complex on the cell surface is to be targeted without specifying the target molecule, a sorting experiment using cells is carried out. A mixed solution of beads and cells is observed using a CCD camera or the like to collect, by aspiration, beads to which the cells are bound. When the number of such beads is large, the beads are dissociated from the cells, and allowed to interact with cells not expressing the target molecule or with cells of non-target lineage, to exclude beads that are thought to be poorly specific. Thereafter, the ligand sequences of the obtained beads are analyzed. The method is applicable to search for molecules that specifically recognize cancer cells, and ligands of membrane proteins such as GPCRs.

In an experiment in which proteins in a solution are used for the screening, not only artificially prepared recombinant proteins, but also proteins whose genes are not modified and which are obtained from a cell extract or the like are used as target molecules. In the former case, the proteins are allowed to interact with a bead library, and then the beads are washed, followed by detecting beads to which the target molecules are bound, based on the activity of fused alkaline phosphatase or the like. In the latter case, the proteins are first labeled with an appropriate amount of a fluorescent labeling agent, and then subjected to a sorting experiment in which beads showing fluorescence activity are sorted.

The OPOB obtained by the present invention is suitable for comprehensive search for ligands, and could be easily used for screening experiments targeting a large number of target molecules at once. Furthermore, since the beads used in the experiment can be reused while maintaining almost the same recognition ability, they are applicable to selective experiments in a plurality of systems.

## Claims

1. A peptide-immobilized bead library having a structure of the following General Formula [I]: 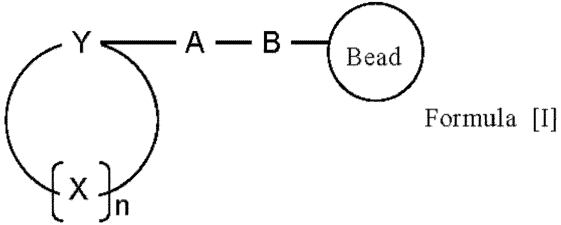 (wherein in General Formula [I], X is an amino acid residue; n is an integer of 4 to 8; Y is a cyclization structure; A is a spacer structure; B is a specific cleavage site-containing structure; and the 4 to 8 amino acid residues of X are each independently randomly selected).

2. The peptide-immobilized bead library according to claim 1, wherein the Y is selected from a disulfide bond, a thioether bond, and an amide bond.

3. The peptide-immobilized bead library according to claim 2, wherein the Y is a structure containing a disulfide bond, and constituted by two cysteine residues.

4. The peptide-immobilized bead library according to claim 2, wherein the Y is a structure containing a thioether bond, and constituted by a homocysteine or cysteine, and an acetyl group.

5. The peptide-immobilized bead library according to any one of claims 1 to 4, wherein the B is a methionine residue.

6. The peptide-immobilized bead library according to any one of claims 1 to 5, wherein the A is a structure containing 3 to 30 atoms constituting the main chain of the A.

7. The peptide-immobilized bead library according to any one of claims 1 to 6, wherein
the 4 to 8 amino acid residues of X are each independently randomly selected from a group of amino acid residues; and
half or more of the amino acid residues constituting the group are amino acid residues other than the 20 kinds of amino acid residues constituting naturally occurring proteins.
